# EUROPEAN PATENT SPECIFICATION

(11) **EP 3 628 305 B1**
(45) Date of publication and mention of the grant of the patent: **22.01.2025**
(21) Application number: 19730104.7
(22) Date of filing: 25.03.2019
(51) Int. Cl.: A61Q 19/00, A61K 8/42, A61K 8/49, A61K 8/9794, A61K 8/73, A61Q 17/00, A61K 8/67, A61K 8/44, A61K 36/899

(54) **HYPOALLERGENIC COSMETIC COMPOSITION**
ANTIALLERGISCHE KOSMETISCHE ZUSAMMENSETZUNG
COMPOSITION COSMÉTIQUE HYPOALLERGÉNIQUE

(30) Priority: 01.08.2018 CN 201810863822
(43) Date of publication of application: 01.04.2020
(73) Proprietor: Yangshengtang (ANJI) Cosmetics Co., Ltd., Huzhou, Zhejiang 313399 (CN)
(72) Inventor: WEN, Yi, HUZHOU, Zhejiang 313399 (CN); WU, Ruixue, HUZHOU, Zhejiang 313399 (CN); ZHENG, Xiaowei, HUZHOU, Zhejiang 313399 (CN); NING, Xinjuan, HUZHOU, Zhejiang 313399 (CN)
(74) Representative: J A Kemp LLP
(86) International application number: PCT/CN2019/079521
(87) International publication number: WO 2020/024598

(56) References cited:
- EP-A1- 1 893 161
- EP-B1- 1 893 161
- CN-A- 101 732 204
- CN-A- 105 708 757
- CN-A- 107 184 411
- DATABASE GNPD [online] MINTEL; 6 June 2018 (2018-06-06), ANONYMOUS: "Hypoallergenic Hydrating Night Cream", XP055740930, retrieved from www.gnpd.com Database accession no. 5722139
- DATABASE GNPD [online] MINTEL; 7 July 2015 (2015-07-07), ANONYMOUS: "Super Sensitive SOS Emergency Care", XP055740954, retrieved from www.gnpd.com Database accession no. 3252729
- DATABASE WPI Week 201683, 1 July 2016 Derwent World Patents Index; AN 2016-50309P, XP002800742
- NN: "CP Oat Avenanthramide Extract", 19 August 2014 (2014-08-19), Canada, pages 1 - 4, XP055740779, Retrieved from the Internet <URL:https://web.archive.org/web/20140819085352if_/http://ceapro.com/wp-content/uploads/2012/09/CPOatAvenanthramideExtract-902-3043-Jun-131.pdf> [retrieved on 20201016]
- NN: "Symrise launches an-itch ingredient", NUTRITION INSIGHT, 17 January 2006 (2006-01-17), pages 1 - 1, XP055740789, Retrieved from the Internet <URL:https://www.nutritioninsight.com/news/symrise-launches-anti-itch-ingredient.html> [retrieved on 20201016]
- RUNA SUR ET AL: "Avenanthramides, polyphenols from oats, exhibit anti-inflammatory and anti-itch activity", ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 300, no. 10, 7 May 2008 (2008-05-07), pages 569 - 574, XP019657186, ISSN: 1432-069X, DOI: 10.1007/S00403-008-0858-X

## Description

### Technical field

The present invention belongs to the field of cosmetics, particularly relates to an anti-allergic cosmetic composition having the function of protecting mast cells, comprising a combination of D-panthenol, allantoin and oat kernel extract, and ingredients commonly used in cosmetic compositions.

### Background art

Due to the deterioration of environment, the increase of life and work stress, and excessive skin cleaning, more and more people suffer from skin barrier damage, and inflammatory symptoms such as erythema, itching and prickling appear on the skin. At the same time, improper use of cosmetics aggravates these symptoms. Therefore, there is an urgent need for cosmetics having obvious anti-inflammatory effects, safety, and no adverse reactions to ease allergy symptoms. As example for ingredients with anti-inflammatory and anti-allergic action on skin can be cited the avenanthramides from oats (RUNA SUR ET AL: "Avenanthramides, polyphenols from oats, exhibit anti-inflammatory and anti-itch activity", ARCHIVES OF DERMATOLOGICAL RESEARCH, vol. 300, no. 10, 7 May 2008, pages 569-574).

Mast cells are widely distributed around microvessels in the skin and visceral mucosa, and contain specific cytoplasmic granules, which store inflammatory mediators. Previous studies have shown that mast cells drive immediate allergies and are also involved in the process of allergic diseases. Increased number and degranulation of mast cells are common pathological phenomena in the lesions of atopic dermatitis and other diseases. In allergic contact dermatitis, mast cells are involved in the activation and migration of antigen presenting cells, and the substances released by their degranulation also mediate the initial process of inflammations. Mast cells express a large number of gE Fc receptors on the surface thereof. In immediate allergies, antigens re-invade and bind to the Fc receptor, and rapidly trigger mast cells to release inflammatory mediators and initiate inflammatory signal transduction cascade. Mast cells release inflammatory mediators such as histamine and tryptase to extracellular space. This process is called as degranulation. The release of inflammatory mediators directly mediates a series of immune inflammatory reactions induced by mast cells.

In current cosmetics, there is a lack of anti-inflammatory and soothing products that can effectively protect mast cells and inhibit the release of inflammatory mediators. Therefore, in view of the deficiencies of existing products, it is necessary to provide a product that can protect mast cells to achieve soothing and anti-inflammatory effects so as to alleviate skin allergies.

### Content of the invention

In an aspect, the present invention relates to a combination of D-panthenol, allantoin and oat kernel extract for use as an anti-allergic composition for the skin; wherein in the anti-allergic composition, the amount of D-panthenol is 0.02-3%, the amount of allantoin is 0.01-1.5%, and the amount of oat kernel extract is 0.2-5%, based on the total weight of the anti-allergic composition.

The anti-allergic cosmetic composition of the invention can repair fragile skin, strengthen skin barrier, effectively protect mast cells, inhibit the release of inflammatory mediators, and thus has good anti-inflammatory and soothing effects. As compared to the use of ingredients alone or the use of conventional anti-inflammatory substances, the combination of D-panthenol, allantoin and oat kernel extract in the anti-allergic cosmetic composition according to the present invention provides significantly improved mast cell protection and anti-inflammatory and soothing effects, indicating that the ingredients in the combination generate synergistic effect.

The D-panthenol, also known as vitamin B5, after being absorbed by the skin in a long-term use, can be transformed into pantothenic acid and synthesized by tissues into coenzyme A, which exists in tissues and is necessary for cell metabolism. Therefore, long-term use of vitamin B5 can improve cell viability, repair damaged skin and enhance skin barrier function. The D-panthenol used in the invention is commercially available, e.g. from DSM or other suppliers.

The D-panthenol is present in the cosmetic composition in an amount of 0.02-3%, preferably 0.05-2%, more preferably 0.1-1%, based on the total weight of the cosmetic composition.

The allantoin can enhance the water absorption capacity of the outermost layer of the skin, improve the hydrophilicity of the stratum corneum protein, and repair the damaged stratum corneum to restore its natural hydrophilic ability. The allantoin used in the present invention is commercially available, e.g. from Clariant or other suppliers.

The allantoin is present in the cosmetic composition in an amount of 0.01 to 1.5%, preferably 0.03-1%, more preferably 0.05-0.5%, based on the total weight of the cosmetic composition.

The oat kernel extract can promote the growth of fibroblasts, inhibit the degradation of hyaluronic acid in the skin, and promote the synthesis of hyaluronic acid; on the other hand, it has anti-irritation, anti-histamine, and UV erythema mitigation characteristics. The oat kernel extract used in the present invention is a pale yellow clear liquid, and it's main ingredient is avenanthramides, which can be obtained by a known extraction technique, for example, it can be obtained by extraction with water and a polyol system. The oat kernel extract is commercially available, e.g. from Symrise or other suppliers.

The oat kernel extract is present in the cosmetic composition in an amount of 0.2-5%, preferably 0.5-4%, more preferably 1-3%, based on the total weight of the cosmetic composition.

The anti-allergic cosmetic composition according to the present invention further comprises ingredients commonly used in cosmetics, their examples include, but are not limited to, active ingredients, vehicles, surfactants, excipients, and any other ingredients known in the art, which are known to those skilled in the art, and their type and amount can be specifically selected as needed.

The active ingredients are any active substances known and commonly used in the art, including, e.g. emollients, moisturizers, skin conditioners, and the like.

Examples of the emollients include, but are not limited to, one or more of glyceryl tri(2-ethylhexanoate), caprylic/capric triglyceride, shea butter, cetyl alcohol, polydimethylsiloxane, pentaerythritol tetra(2-ethyl hexanoate), olive oil, grape seed oil, meadowfoam seed oil, avocado oil, corn oil, squalane, dioctyl carbonate, isopropyl myristate, hydrogenated polydecene, sunflower seed oil, isohexadecane, jojoba seed oil, lanolin, paraffin wax, microcrystalline wax, beeswax, and the like. In the compositions of the present invention, the emollients account for about 1-50% of the total weight of the commonly used ingredients.

Examples of the moisturizers include, but are not limited to, one or more of birch juice, glycerin, betaine, glycereth-26, trehalose, sucrose, propylene glycol, 1,2-pentanediol, mannitol, rhamnose, raffinose, erythritol, xylitol, urea, PEG-8, PEG-32, methyl gluceth-10, methyl gluceth-20, PEG/PPG-17/6 copolymer, sodium polyglutamate, hydrolyzed sclerotium gum, pullulan, tremella polysaccharide, sodium polyglutamate, glyceryl glucoside, PPG-10 methyl glucose ether, PPG-20 methyl glucose ether, and the like. In the compositions of the present invention, the moisturizers account for about 1-95% of the total weight of the commonly used ingredients.

Examples of the vehicles include, but are not limited to, diluents, dispersants, carriers, or the like. All the vehicles are known in the art, and their type and amount can be selected by those skilled in the art as needed. Examples include, but are not limited to, water, ethanol, dipropylene glycol, butylene glycol, and the like. In the compositions of the present invention, the vehicles account for about 1-20% of the total weight of the commonly used ingredients.

The skin conditioners may be of any types known in the art and have moisturizing, anti-wrinkle, anti-freckle, anti-acne, oil-control and other functions.

Examples of the skin conditioners include, but are not limited to, one or more of phytosteryl/octyldodecyl lauroyl glutamate, hydrolyzed sodium hyaluronate, acetyl phytosphingosine, curcuma longa, ceramide 2, ceramide 3, cholesterol, kojic acid, ascorbic acid, ascorbyl glucoside, arbutin, tranexamic acid, nicotinamide, birch bark extract, acetyl sphingosine, resveratrol, petrocarpus marsupium bark extract, Coleus forskolini, Fructus piperis extract, ubiquinone, bisabolol, ascorbyl tetraisopalmitate, pyridoxine dicaprylate, pyridoxine dipalmitate, retinyl palmitate, and the like. Generally, in the cosmetic compositions of the present invention, the skin conditioners account for about 0.05-50% of the total weight of the commonly used ingredients.

The surfactants may be any type of surfactants commonly used in cosmetics for reducing the surface tension of the interface to achieve the purpose of cleaning, emulsifying, and stabilizing the system. For example, examples of the surfactants include, but are not limited to, one or more of fatty acid soaps (e.g. sodium laurate, sodium palmitate, etc.), higher alkyl sulfates (e.g. sodium lauryl sulfate, etc.), N-acyl sarcosine (e.g. sodium N-lauroyl sarcosinate, etc.), higher fatty acid amide sulfonates (e.g. sodium lauryl methyl taurate, etc.), alkylbenzene sulfonates, higher fatty acid ester sulfates (e.g. hardened coconut oil fatty acid sodium glyceryl sulfate, etc.), N-acyl glutamate, lauryl dimethylaminoacetic acid betaine, alkyl betaines, amido betaines, sorbitan fatty acid esters (e.g. sorbitan monooleate, sorbitan monoisostearate, sorbitan monolaurate, sorbitan monopalmitate, sorbitan monostearate, sorbitan sesquioleate), glyceryl polyglyceryl fatty acid esters (e.g. glyceryl monoerucate, glyceryl sesquioleate, glyceryl monostearate, glyceryl monostearic acid malate, etc.), PEG-fatty acid esters (e.g. PEG-distearate, ethylene glycol distearate etc.), PEG-alkyl ethers (e.g. PEG-2-octyl dodecyl ether, etc.), sucrose fatty acid esters, and the like. The amount of the surfactants in the compositions of the present invention is known in the art, and is typically about 0.05-50% of the total weight of the commonly used ingredients.

The excipients include, but are not limited to, emulsifiers, thickeners, preservatives, perfumes, pH regulators, and the like.

Examples of the emulsifiers include, but are not limited to, PEG-60 hydrogenated castor oil, glyceryl stearate/PEG-100 stearate, sorbitan olivate, steareth-21, PPG-13-decyltetradeceth-24, cetearyl glucoside, cetearyl glucoside, polyglyceryl-10 stearate, polyglyceryl-10 myristate, polyglyceryl-10 dioleate, and the like. Generally, in the compositions of the present invention, the emulsifiers account for 0.5-10% of the total weight of the commonly used ingredients.

Examples of the thickeners include, but are not limited to, carbomer, xanthan gum, SIMUGEL EG, gum arabic, polyethylene glycol-14M, polyethylene glycol-90M, succinoglycan, hydroxyethyl cellulose, hydroxypropyl cellulose, and the like. Generally, in the compositions of the present invention, the thickeners account for about 0.1-10% of the total weight of the commonly used ingredients.

Examples of the preservatives include, but are not limited to, methylparaben, propylparaben, phenoxyethanol, benzyl alcohol, phenylethyl alcohol, potassium sorbate, sodium benzoate, chlorphenesin, and the like, and other antiseptic synergists, such as pentanediol, hexanediol, caprylyl glycol, p-hydroxyacetophenone, and the like. Generally, in the compositions of the present invention, the preservatives account for about 0.01-2% of the total weight of the commonly used ingredients.

Examples of the pH regulators include, but are not limited to, citric acid, sodium citrate, arginine, sodium hydroxide, potassium hydroxide, and the like. Their type and amount can be specifically selected by those skilled in the art as needed.

The cosmetic compositions of the present invention can be prepared by any suitable methods known in the art. For example, the preparation can be carried out according to a process known in the art using a dissolution tank, an emulsifying pot, a disperser, a transfer pump and other equipment which are commonly used in the field of cosmetics.

For example, a water-soluble substance may be charged into an aqueous phase dissolution kettle, and an oil-soluble substance may be charged into an oil phase dissolution kettle, and the kettles are heated to about 80°C, respectively, wherein the raw materials which are easy to cake may be pre-dispersed first by a disperser; upon the completion of the dissolution, the oil phase and the aqueous phase are delivered to an emulsifying pot for homoemulsification for about 5-15 mins; upon the completion of the emulsification, the material temperature is lowered to normal temperature, optionally perfumes, preservatives and other ingredients are added, and, if desired, the pH of the product is regulated; after relevant test indexes are qualified, it can be canned and shipped.

The above preparation process is merely an example, and those skilled in the art can increase, decrease or adjust preparation process according to the requirements of formulations to obtain spray, lotion, ointment, cream, gel or other formulations.

### Examples

The present invention is further described in detail with reference to examples below. It should be understood that the examples described herein merely illustrate the technical solution of the present invention and are not intended to limit the scope of the present invention.

### Example 1: Preparation of anti-allergic spray A

In this example, an anti-allergic spray A having the following ingredients was prepared.

| Ingredients | Spray A ( wt.% ) |
|---|---|
| Water | 96.6 |
| Panthenol | 0.5 |
| Allantoin | 0.2 |
| Oat kernel extract | 1.3 |
| Glycerin | 0.8 |
| Betaine | 0.6 |

The preparation procedure of spray A was as follows:
(1) heating water to 80°C, adding allantoin, glycerin, panthenol and betaine in turn, mixing, stirring and dissolving uniformly;
(2) cooling the mixture to 40°C, adding oat kernel extract, and discharging. The resulting spray A was a pale yellow clear liquid.

Example 2 (comparative example): Preparation of control sprays B, C, D and E In this example, control sprays B, C, D, and E having the following ingredients were prepared using a procedure similar to the above, and all of the control sprays did not contain a combination of panthenol, allantoin and oat kernel extract. The resulting spray B was a colorless clear liquid, and the sprays C, D, and E were pale yellow clear liquids.

| Ingredients ( wt.% ) | Spray B | Spray C | Spray D | Spray E |
|---|---|---|---|---|
| Water | 96.6 | 96.6 | 96.6 | 96.6 |
| Panthenol | 1.6 | - | - | 0.4 |
| Allantoin | 0.4 | - | 0.4 | - |
| Oat kernel extract | - | 2.0 | 1.6 | 1.6 |
| Glycerin | 0.8 | 0.8 | 0.8 | 0.8 |
| Betaine | 0.6 | 0.6 | 0.6 | 0.6 |

### Example 3: Effect of sprays A-E on allergic reaction of RBL-2H3 cells

The effect of the prepared sprays A-E on allergic reaction of cells was tested according to following methods.

Experimental principle: Compound 48/80 is a tool drug to induce mast cell degranulation, the mechanism is that it acts on mast cell membrane and induces an increase of intracellular calcium ions to change the amount of second messengers cAMP and cGMP, resulting in mast cell degranulation and histamine release.

Experimental materials: RBL-2H3 cell line was provided by the animal center of Zhejiang Academy of Medical Sciences; the test samples included 5 sprays A-E prepared above; the reagents included fetal bovine serum, RPM1640 medium, Tyrode's salt (mainly composed of sodium chloride, phosphate, calcium chloride, glucose, etc.), Compound 48/80 and histamine Elisa kit.

### Experimental procedures:

| Preparation of Compound | 1.0 mg of 48/80 powder was weighed and diluted with 1 mL of Tyrode's solution, and 50 uL of the dilution was diluted to |
|---|---|
| 48/80 solution | 1000 uL, i.e. 48/80 solution at a concentration of 50 ug/mL, and the solution should be used right after it was ready. |
| Preparation of Tyrode's solution | 9.6 g of Tyrode's salt was diluted with 80 mL of sterilized deionized water, constant-volumed to 100 mL to prepare 10 times concentrate of Tyrode's solution, and autoclaved. |
| Pre-treatment of water sample | Sterilization and pH determination were carried out ahead, 900uL of water sample was mixed with 1000 uL of 10 times Tyrode's solution, ready for use. |
| Cell treatment | RBL-2H3 cells in logarithmic growth phase were spreaded to the bottom of a culture flask and digested with a digestive juice containing 0.25% trypsin and 0.03% EDTA, centrifuged at 1200 rpm for 5 mins; resuspended with 1 time Tyrode's solution and counted, and diluted to 1×10⁴/mL; centrifuged again at 1200 rpm for 5 mins to remove the supernatant. |
| Water sample grouping and experiments | Model group: 700uL of cell suspension was centrifuged and resuspended with Tyrode's solution, stood at 37°C for 30 mins; sample group: 700uL of cell suspension was centrifuged and resuspended with sample solution, stood at 37°C for 30 mins; the sample was mixed uniformly and dispensed into 3 tubes, 200uL per tube; the model group and the 5 spray sample groups: 5uL of 48/80 solution was added to the cell suspension at a final concentration of 10 ug/mL, and stood at 37°C for 20 mins. |
| Histamine determination | The above groups of samples were cooled in an ice box for 10 mins to terminate the reaction. The samples after termination of the reaction were centrifuged (4°C, 1500 rpm, 30 mins) to precipitate the cells at the bottom of EP tube, and the supernatants were transferred to new centrifuge tubes. The precipitated cells were resuspended with 250 µL of 1 time Tyrode's solution, heated in a 90°C water bath, taken out after 5 mins and immediately placed in an ice box, after the samples were completely cooled, they were taken out and heated again in a 90°C water bath, and frozen-thawed repeatedly several times to break cells. The supernatants and the cell solutions were diluted 10 times, and the stock solutions were stored in a refrigerator at -20°C. The diluted samples were determined for histamine using an imported histamine kit and calculated. The histamine release was |
| | calculated using the following formula: histamine release (%) = extracellular histamine concentration/(extracellular histamine concentration + intracellular histamine concentration) × 100% |

Experimental results: The experimental results are summarized in Table 1 below.

**Table 1: Test results of histamine release in cell model group**

| | Release % | P value (vs. model) | P value (vs. spray A) |
|---|---|---|---|
| Model group | 66.4±8.7 | - | 0.000 |
| Spray A | 36.6±9.2 | 0.000 | - |
| Spray B | 50.0±6.5 | 0.001 | 0.010 |
| Spray C | 45.5±6.0 | 0.001 | 0.005 |
| Spray D | 47.4±7.8 | 0.000 | 0.025 |
| Spray E | 49.6±8.1 | 0.000 | 0.039 |

The results of the above cell experiments showed that spray A in the scope of the present invention significantly reduced histamine release as compared to control sprays B-E, indicating that the combination of panthenol, allantoin and oat kernel extract had synergistic effect on inhibition of histamine release.

Example 4: Protective effect of sprays A-E on mast cells of zebrafish larvae The protective effect of sprays A-E prepared above on mast cells of zebrafish larvae was measured according to following methods.

### Experimental animals: Zebrafish 5dpf (days post fertilization)

Experimental reagents: Substance P (SP, molecular formula C63H98N18O13S) is a neuropeptide, belonging to the tachykinin neuropeptide-family. SP can induce mast cell degranulation, cause rapid release of histamine, tryptase and other mediators, and directly mediate a series of immunoinflammatory reactions caused by mast cells.

### Index of evaluation: Tryptase release

Experimental samples: Sprays A-E prepared above, and 60 ug/ml ketotifen used as a positive control.

Experimental methods: Wild-type zebrafish embryos were collected and cultured in a 28.5°C incubator with E3 buffer (for larvae culture, containing 5 mM NaCl, 0.17 mM KCI, 0.33 mM CaCl₂, 0.33 mM MgS0₄, supplemented with water to the final volume) to 5 dpf (days post fertilization), the buffer was changed every day, and the zebrafish 5 dpf was randomly transferred to 48-well cell culture plates in groups of 10 tails per well, 4 duplicate wells per group, grouped as follows:
Model group Msp: pure water + 15 µg/ml SP
Positive group Tsp: 60 µg/ml ketotifen + 15 µg/ml SP
Sample group to be tested X_{SP}: spray sample to be tested + 15 µg/ml SP
wherein, positive drug ketotifen was formulated into a mother liquor with DMSO and stored at -20°C, and it was formulated into 60 µg/ml fluid with pure water when using in the experiment.

Corresponding to the above degranulation groups with SP, a negative control group without SP was established, 4 duplicate wells per group; corresponding to the SP-induced degranulation group and the negative control group without SP, a background control group (without zebrafish larvae) was established, 2 duplicate wells per group, and the E3 buffer involved in each group of wells was absorbed, and 250 µl of a solution corresponding to each group was added, and reacted in darkness in 28.5°C incubator for 60 mins; after 60 mins, 200 µl of the supernatant of each group was placed in a 96-well cell culture plate, and added with enzyme reaction substrate BAPNA (trypsin substrate) respectively to reach a concentration of 400 µg/ml, and the 96-well plate was covered in darkness and placed in a 28.5°C incubator, and reacted for 2 hours. The full plate was measured for light absorption value at 405 nm after 2 hours. The value reflects tryptase release in the mast cells of zebrafish. The protection rate of mast cells to be tested was calculated based on the following formula: protection rate of mast cells of sample to be tested= [(Msp-Msp.bg)-(RO-RObg)]-[(Xsp-Xsp.bg)-(X-Xbg)]/(Msp-Msp.bg)-(RO-RObg)×100% wherein:
Mₛₚ-M_{sp.bg} represents the absorbance of the SP-induced degranulation group (i.e. model group) minus the absorbance of its corresponding background control group (without zebrafish larvae);
RO-RO_{bg} represents the absorbance of the ultra-clean water group without SP (i.e. the negative control group of the model group) minus the absorbance of its corresponding background control group;
Xₛₚ-X_{sp.bg} represents the absorbance of the sample group of SP-induced degranulation minus the absorbance of its corresponding background control group;
X-X_{bg} represents the absorbance of the sample group without SP minus the absorbance of its corresponding background control group.

The experimental results were summarized in Table 2 below.

**Table 2: Experimental results of Zebrafish larvae mast cell protection model effects**

| Group | Xsp-Xsp•bg | X-Xbg | Mast cell protection rate |
|---|---|---|---|
| Model group | 0.061 | 0.006 | - |
| Positive group | 0.006** | 0.003 | 95.45% |
| Spray A | 0.002** | 0.001 | 97.73% |
| Spray B | 0.220** | 0.013 | 0 |
| Spray C | 0.028** | 0.018 | 82.27% |
| Spray D | 0.016** | 0.009 | 88.18% |
| Spray E | 0.028** | 0.022 | 88.64% |

| | | | |
|---|---|---|---|
| Note: * indicates p < 0.05, ** indicates p < 0.01 (as compared to the model group); if the mast cell protection rate is greater than 100%, it is regarded as 100%, and if the mast cell protection rate is less than 0, it is regarded as 0. | | | |

The results in Table 2 showed that spray A in the scope of the present invention had a significantly improved mast cell protection effect as compared to the model group and the control sprays B-E, indicating that the combination of oat kernel extract, panthenol and allantoin had synergistic effect in protecting mast cell degranulation.

Example 5: Anti-inflammatory effect of sprays A-E in mouse models The anti-inflammatory effect of sprays A-E in mouse models were measured according to following methods.

### Experiments on irritant contact dermatitis

Irritant contact dermatitis (ICD) is caused by the strong irritation (such as strong acid and alkali) or toxicity of contactant itself. Its histopathological manifestations include vasodilation, plasma exudation, and infiltration of leukocytes in blood into local tissues to cause an inflammatory reaction. It is generally believed that ICD is a non-immune inflammatory reaction. In this experimental model, chemical reagents were used to stimulate abdominal nude skin of mice (10% sodium dodecyl sulfate, SDS), causing damage of skin barrier and aggravating local inflammatory reaction of the skin. The inflammatory factor index was used to determine whether the model was established for later anti-inflammatory efficacy evaluation experiments.

Experimental animals: ICR mice, 20-25 g, male.

Experimental materials: 10% sodium dodecyl sulfate solution (SDS), 1% hydrocortisone ointment, depilatory paste.

Experimental methods: ICR mice were randomly divided into groups according to body weight, 12 mice in each group, including normal group, model group, positive group, and spray test groups. At 1 day before modeling, the mice in each group were treated with depilatory paste to remove about 2*2 cm area of the hair in the abdomen of the mice. The model group, the positive group, and the test groups: on the day of modeling, 50 µL of a 10% sodium dodecyl sulfate solution was pipetted and evenly applied to the nude skin of the mice for 5 days. After modeling: the positive group was treated with 1% hydrocortisone ointment 3 times a day continuously for 2 days; the model group was not treated. Each test group solution was administered in the same manner as the positive group. The mice in the normal group were treated by the same method to remove the abdominal hair, fed for 7 days and compared. On the 7th day of the experiment, the skin condition of each group of experimental mice was observed, and the serum of mice was collected for determining the amount of inflammatory factor IL-1a.

### Index of evaluation: Determination of the amount of inflammatory factor IL-1a in serum

Index explanation: Acute barrier dysfunction can activate MyD pathway and increase the synthesis of inflammatory factor IL-1a downstream of MyD pathway, which is repeatedly observed in the inflammatory state of the skin. In this experiment, the feasibility of irritant dermatitis caused by 10% SDS was observed by comparing model group, positive group and normal group, and tested by the treatment with a positive drug hydrocortisone.

### Experiments on allergic contact dermatitis

Cosmetic allergy is commonly referred to as cosmetic allergic contact dermatitis, which is as high as 30.0-46.2% of cosmetic dermatitis. Cosmetic allergic contact dermatitis is often more severe than irritant dermatitis, and cross-allergic reactions occur, and treatment is relatively difficult. Therefore, 2,4-dinitrofluorobenzene (DNFB) was used to sensitize and stimulate mice to establish allergic contact dermatitis model (ACD), and 1% hydrocortisone ointment was administered as a positive control, and efficacy evaluation experiments were carried out in the test groups.

Experimental animals: ICR mice, 20-25 g, male.

Experimental materials: DNFB acetone/olive oil (4:1) solution, 1% hydrocortisone ointment, depilatory paste.

Experimental methods: ICR mice were randomly divided into groups according to body weight, 12 mice in each group, including normal group, model group, positive group, and spray test groups. At 1 day before modeling, the mice in each group were treated with depilatory paste to remove about 2*2 cm area of the hair in the abdomen of the mice. On the day of modeling, the mice in the model group, the positive group and the test groups were sensitized by applying 100 µL of a 5% DNFB acetone/olive oil solution evenly to the nude skin of the mice continuously for 2 days. On the 5^{th} day, the mice were stimulated by applying 30 µL/ear of 1% DNFB acetone/olive oil solution evenly to the inner and outer auricular surfaces of both ears in the mice to establish an allergic contact dermatitis model. The mice in the normal group were treated with acetone/olive oil (4:1) solution in the abdomen and ears.

After 16 hours of stimulation, the drug was administered. For the test groups, 30 µL/ear of the sample was pipetted and evenly applied to the inner and outer auricular surfaces with cotton sticks, three times a day, morning, noon and night, continuously for 2 days. The model group was sprayed with distilled water, and the positive group was sprayed with 1% hydrocortisone ointment. Whole blood was collected 4 h later after the last administration, and the mice were executed.

### Index of evaluation

Ear thickness index: After execution, the thickness of mice's ears was measured, and the average thickness of both ears was obtained.

Ear weight index: After execution, each mouse's ears were collected by a perforator (Deli, 8mm diameter), and the total weight was weighed.

Inflammatory index determination: The serum of mice was collected, and the amount of inflammatory factor IFN-γ in the ear tissues of the mice was detected by ELISA kit.

Index explanation: Th1/Th2 imbalance is an important link or initiating factor in the pathogenesis of allergic diseases. Normally, Th1 cells and Th2 cells are in dynamic equilibrium, and inhibit each other by secreting their own cytokines (Th1 cytokines such as IFN-r, Th2 cytokines such as IL-4). Th1 cells produce IFN-Y, TNF-α, IL-2 and the like, causing contact allergy; Th2 cells produce IL-4, IL-5, IL-10, IL-13 and the like, inhibiting contact hypersensitivity.

The experimental results were summarized in Table 3 below.

**Table 3: Anti-inflammatory effect test in mouse models**

| | Ear thickness (mm) | | Ear weight (mg) | | Th1/Th2 | | INF-γ (pg/ml) | | IL-1a (pg/ml) | |
|---|---|---|---|---|---|---|---|---|---|---|
| | Mean value | P value | Mean value | P value | Mean value | P value | Mean value | P value | Mean value | P value |
| Model group | 0.39 | - | 22.08 | - | 2.53 | - | 99.2 | - | 30.54 | - |
| Normal group | 0.18 | 0.00 | 8.88 | 0.00 | 1.21 | 0.00 | 72.2 | 0.02 | 20.85 | 0.00 |
| Positive group | 0.27 | 0.00 | 14.17 | 0.00 | 1.36 | 0.01 | 82.6 | 0.01 | 19.17 | 0.00 |
| Spray A | 0.27 | 0.00 | 13.71 | 0.00 | 1.07 | 0.00 | 69.3 | 0.00 | 19.40 | 0.00 |
| Spray B | 0.32 | 0.02 | 19.33 | 0.01 | 1.80 | 0.05 | 82.3 | 0.01 | 24.36 | 0.03 |
| Spray C | 0.30 | 0.00 | 17.67 | 0.01 | 1.58 | 0.03 | 80.2 | 0.03 | 24.46 | 0.04 |
| Spray D | 0.30 | 0.00 | 17.83 | 0.01 | 1.59 | 0.04 | 81.3 | 0.05 | 24.49 | 0.05 |
| Spray E | 0.31 | 0.00 | 19.29 | 0.02 | 1.59 | 0.04 | 80.6 | 0.04 | 24.37 | 0.04 |

The above results showed that the anti-inflammatory effect of spray A in the scope of the invention was significantly better than that of sprays B-E in all indexes, indicating that the combination of oat kernel extract, panthenol and allantoin had synergistic effect on the relief of contact dermatitis in mice.

### Example 6: Preparation of anti-allergic toner

This example provided an anti-allergic toner composition comprising the following ingredients:

| Ingredients | Amount (wt% ) |
|---|---|
| Birch juice | 75.25 |
| Panthenol | 1.2 |
| Allantoin | 0.3 |
| Oat kernel extract | 3 |
| Glycerin | 5 |
| Betaine | 3 |
| Glycereth-26 | 0.5 |
| phytosteryl/octyldodecyl lauroyl glutamate | 1 |
| Butanediol | 5 |
| Pentanediol | 2 |
| Glyceryl tri(2-ethylhexanoate) | 2 |
| PEG-60 hydrogenated castor oil | 1 |
| Citric acid | 0.1 |
| Arginine | 0.1 |
| Methyl hydroxybenzoate | 0.15 |
| Phenoxyethanol | 0.4 |

The toner composition was prepared as follows:
1. The birch juice was heated to 80°C, and citric acid, panthenol, allantoin, 3 parts of glycerin, methyl hydroxybenzoate, butanediol, pentanediol and betaine were added in turn, mixed, stirred and dissolved uniformly;
2. Phytosteryl/octyldodecyl lauroyl glutamate, glyceryl tri(2-ethylhexanoate), 2 parts of glycerin, glycereth-26 and PEG-60 hydrogenated castor oil were heated to 80°C, stirred uniformly, and then added to the mixture obtained in step 1;
3. the resultant was cooled to 40°C, added with oat kernel extract and phenoxyethanol in turn, the pH was regulated with arginine, and the product was discharged.

The toner exhibited excellent anti-allergic, soothing and other effects in skin care.

### Example 7: Preparation of anti-allergic lotion

This example provided an anti-allergic lotion composition comprising the following ingredients:

| Ingredients | Amount (wt%) |
|---|---|
| Water | 78.1 |
| Panthenol | 1.5 |
| Allantoin | 0.5 |
| Oat kernel extract | 3 |
| Glyerin | 5 |
| Hydrolyzed sodium hyaluronate | 1 |
| Dipropylene glycol | 3 |
| Caprylic/capric triglyceride | 3 |
| Shea butter | 1 |
| Carbomer | 0.15 |
| Xanthan gum | 0.1 |
| Cetyl alcohol | 1 |
| Glyceryl stearate/PEG-100 stearate | 1 |
| Glyceryl tri(2-ethylhexanoate) | 1 |
| Phenoxyethanol | 0.4 |
| Methyl hydroxybenzoate | 0.1 |
| Arginine | 0.15 |

The above anti-allergic lotion composition was prepared as follows:
1. Aqueous phase: water, glycerin, panthenol, allantoin, hydrolyzed sodium hyaluronate, dipropylene glycol, xanthan gum, carbomer, and methyl hydroxybenzoate were mixed and heated to 80°C, stirred and dissolved uniformly;
2. Oil phase: cetyl alcohol, glyceryl stearate/PEG-100 stearate, caprylic/capric triglyceride, glyceryl tri(2-ethylhexanoate), and shea butter. The raw materials were heated to 80°C, stirred and dissolved uniformly;
3. The aqueous phase and the oil phase were mixed and homoemulsified for 5 mins, upon the completion of the emulsification, arginine dissolved in water was added, stirred for 15 mins, cooled to 40°C, and added with oat kernel extract and phenoxyethanol in turn.

The lotion exhibited excellent anti-allergy, soothing and other effects in skin care.

### Example 8: Preparation of anti-allergic facial cream

This example provided an anti-allergic facial cream composition comprising the following ingredients:

| Ingredients | Amount (wt% ) |
|---|---|
| Water | 75.15 |
| Panthenol | 1 |
| Allantoin | 0.6 |
| Oat kernel extract | 2 |
| Glyerin | 6 |
| Hydrolyzed sodium hyaluronate | 0.5 |
| Glycereth-26 | 2 |
| Xanthan gum | 0.1 |
| Glyeryl caprylate/caprate | 2 |
| Shea butter | 1 |
| Cetyl alcohol | 1 |
| Glyceryl stearate / PEG-100 stearate | 1.5 |
| SIMUGEL EG | 1 |
| Pentaerythritol tetra(2-ethyl hexanoate) | 3 |
| Polydimethylsiloxane | 2 |
| Methyl hydroxybenzoate | 0.2 |
| Propyl hydroxybenzoate | 0.1 |
| Carbomer | 0.1 |
| Phenoxyethanol | 0.4 |
| Caprylyl glycol | 0.05 |
| arginine | 0.3 |

The above anti-allergic facial cream composition was prepared as follows:
1. Aqueous phase: water, glycerin, panthenol, allantoin, hydrolyzed sodium hyaluronate, glycereth-26, xanthan gum, carbomer, and methyl hydroxybenzoate;
2. Oil phase: cetyl alcohol, caprylate/caprate, pentaerythritol tetra(2-ethyl hexanoate), glyceryl stearate / PEG-100 stearate, shea butter, polydimethylsiloxane, propyl hydroxybenzoate. The raw materials were heated to 80°C, stirred and dissolved uniformly;
3. The aqueous phase and the oil phase were mixed and homoemulsified for 5 mins, upon the completion of the emulsification, arginine dissolved in water was added, stirred for 15 mins, cooled to 40°C, and added with oat kernel extract, phenoxyethanol and caprylyl glycol in turn.

The facial cream exhibited excellent anti-allergy, soothing and other effects in skin care.

The technical solutions of the above examples were preferred embodiments of the present invention. In addition, the soothing and anti-sensitive composition of the present invention can be applied to various cosmetics such as essence, BB cream, sunscreen and so on.

## Claims

1. A combination of D-panthenol, allantoin and oat kernel extract for use as an anti-allergic composition for the skin;
wherein in the anti-allergic composition, the amount of D-panthenol is 0.02-3%, the amount of allantoin is 0.01-1.5%, and the amount of oat kernel extract is 0.2-5%, based on the total weight of the anti-allergic composition.

2. The combination for use according to claim 1, wherein the amount of D-panthenol is 0.05-2%, the amount of allantoin is 0.03-1%, and the amount of oat kernel extract is 0.5-4%.

## Patentansprüche

1. Kombination aus D-Panthenol, Allantoin und Haferkernextrakt für die Verwendung als eine antiallergische Zusammensetzung für die Haut;
wobei in der antiallergischen Zusammensetzung die Menge an D-Panthenol 0,02-3 % beträgt, die Menge an Allantoin 0,01-1,5 % beträgt und die Menge an Haferkernextrakt 0,2-5 % beträgt, auf Grundlage des Gesamtgewichts der antiallergischen Zusammensetzung.

2. Kombination für die Verwendung nach Anspruch 1, wobei die Menge an D-Panthenol 0,05-2 % beträgt, die Menge an Allantoin 0,03-1 % beträgt und die Menge an Haferkernextrakt 0,5-4 % beträgt.

## Revendications

1. Combinaison de D-panthénol, d'allantoïne et d'extrait de noyau d'avoine pour utilisation en tant que composition anti-allergénique pour la peau ;
dans laquelle, dans la composition anti-allergénique, la quantité de D-panthénol est de 0,02 à 3 %, la quantité d'allantoïne est de 0,01 à 1,5 %, et la quantité d'extrait de noyau d'avoine est de 0,2 à 5 %, sur la base du poids total de la composition anti-allergénique.

2. Combinaison pour utilisation selon la revendication 1, dans laquelle la quantité de D à panthénol est de 0,05 à 2 %, la quantité d'allantoïne est de 0,03 à 1 %, et la quantité d'extrait de noyau d'avoine est de 0,5 à 4 %.
